# EUROPEAN PATENT APPLICATION

(11) **EP 4 483 897 A1**
(43) Date of publication of application: **01.01.2025**
(21) Application number: 23461615.9
(22) Date of filing: 30.06.2023
(51) Int. Cl.: A61K 39/108, A61P 31/04, C07K 14/245, C07K 16/12

(54) **IMMUNIZING AND/OR THERAPEUTIC VACCINE AND PREPARATION FOR USE IN PREVENTION AND/OR TREATMENT OF COLIBACTEROSIS IN PIGLETS**

(71) Applicant: Zaklad Badawczo-Wdrozeniowy Osrodka Salmonella "Immunolab" Sp. z o.o., 80-822 Gdansk (PL)
(72) Inventor: LIEDER, Dorota, 83-050 Babidol (PL); GLOSNICKI, Krzysztof, 83-050 Babidol (PL)
(74) Representative: JD&P Patent Attorneys Joanna Dargiewicz & Partners

(57) **Abstract**

The subject matter of the present invention is a vaccine for use in the prevention and/or treatment of colibacterioses in piglets, which contains a mixture of four strains of whole inactivated Escherichia coli bacteria, strains obtained from animals with symptoms of either post-weaning diarrhea or edema disease, and the Stx2eB protein with the sequence **SEQ ID NO: 1** and/or **SEQ ID NO: 3** and/or **SEQ ID NO: 5.**

The subject matter of the invention is also an immunizing and/or therapeutic preparation against colibacterioses in piglets, which contains the above-defined vaccine for administration by injection and chicken IgY antibodies to E. coli strains having O:139, O:149, F:4, F18, F5 antigens, for oral administration.

## Description

The present invention relates to a vaccine and an immunizing and/or therapeutic preparation for use in the prevention and/or treatment of colibacterioses in piglets, and a method of vaccine production.

There are many strains of *E coli* which form part of the physiological intestinal flora of humans and warm-blooded animals. Among the *E coli* species, however, it is possible to distinguish pathogenic strains that cause infections in both animals and humans. *E coli* bacteria that cause pathological conditions usually possess fimbriae (F antigen) allowing bacteria to attach to host cells, produce exotoxins and/or endotoxins, and can produce an envelope (K antigen) with antiphagocytic properties.

From the pig welfare point of view, among the most significant subspecies are enterotoxigenic *E* coli strains (so-called ETEC), which can cause such disease entities as neonatal diarrhea/neonatal colibacteriosis in piglets (up to about 3rd-4th day of life), adaptive colibacteriosis (period of a temporary immunity decrease, i.e. between the 2nd and the 4th week of life), post-weaning diarrhea (PWD, 1st-2nd week after weaning the piglets from the sow) or edema disease (ED).

Direct and indirect losses generated by diarrhea are one of the most serious economic problems of the piglet rearing period. Post-weaning diarrhea (PWD) and edema disease (ED) are currently the most serious economic problems in pig livestock production globally. Researchers estimate that in Europe, 15-23% of all piglets suffer from intestinal problems, causing significant losses in turnover for breeders.

The magnitude of the problem is compounded by current regulations banning the use of antibiotics for metaphylaxis and new bans on the use of zinc oxide in feed additives as an adjuvant to antibiotic treatment during bacterial infections. The most commonly used antibiotics for the PWD treatment are tetracycline and colistin. However, colistin is considered the last therapeutic option for treating infections caused by multidrug-resistant Gram-negative bacteria such as *Pseudomonas aeruginosa, Acinetobacter baumannii, Klebsiella pneumoniae,* and Enterobacter spp. Due to the results of studies conducted over the past few years that show the emergence of colistin-resistant *E coli* strains isolated from pigs, new European recommendations have been made to withdraw drugs containing colistin for the PWD treatment (Updated advice on the use of colistin products in animals within the European Union: development of resistance and possible impact on human and animal health, EMA/CVMP/CHMP/231573/2016).

Zinc oxide is commonly added to feeds to prevent infections and aid antibiotic treatment during bacterial infections. However, the danger of zinc oxide contaminating the environment has been identified after alarming research results indicating a correlation between high doses of zinc in feed and the incidence of bacterial resistance. Due to the conclusions drawn that the use of zinc oxide may promote the selection of zinc-resistant bacteria, the European Union Parliament decided to withdraw zinc oxide from use in feed (Directive 2001/82/EC of the European Parliament and of the Council of 26.6.2017; Press Office Press release Committee for Medicinal Products for Veterinary Use (CVMP) Meeting of 14-16 March 2017, EMA/CVMP/147249/2017).

International patent application WO14065210 A1 relates to a swine edema disease vaccine containing a fusion protein in which Stx2eB and a polypeptide having a coiled-coil forming unit or multimer of the fusion protein are combined.

Korean patent application KR20200032198 relates to a Stx2eA2-Stx2eB composition as a vaccine containing a recombinant Stx2ea2-Stx2eB5 protein for the prevention or treatment of pathogenic *Escherichia coli* in pigs.

International patent application WO17094793 A1 relates to an antigenic vaccine with enhanced immunogenicity that contains a fusion protein comprising an antigenic peptide and an adjuvant protein, wherein the adjuvant protein comprises two or more proteins selected from the group consisting of Shiga toxin 2e subunit B (Stx2eB), *Escherichia coli* heat-labile toxin subunit B (LTB) and cholera toxin subunit B (CTB).

Korean patent application KR20180137866 relates to a vaccine composition for preventing or alleviating the symptoms of pathogenic *E.* coli infectious diseases, wherein the vaccine composition comprises: (1) a Stx2eB-STb fusion protein containing the pathogenic B subunit of *E. coli* Shiga toxin (Stx2eB) and the pathogenic heat-stable enterotoxin b (STb) toxoid of *E. coli* or its functional equivalent; and (2) a Stx2eB-LTb fusion protein containing pathogenic *E. coli* toxoid Stx2eB and the heat-labile B subunit (LTb) of pathogenic *E. coli* enterotoxin, as antigens.

Korean patent application KR2018015647 relates to a vaccine composition containing a recombinant protein: a C-terminal fragment of Stx2EA combined with a Stx2EB pentamer, as an antigen for the prevention or treatment of swine edema disease.

Korean patent application KR20190136134 relates to a vaccine composition with Stx2EA-C-Stx2EB-F4-F18, which contains a C-terminal fragment of the Stx2EA-Stx2eB5 recombinant protein and inactivated F4+ enterotoxigenic *E coli* and F18+ enterotoxigenic *E coli* cells, for the prevention or treatment of pathogenic *Escherichia coli* in pigs.

Chinese patent application CN104593397 relates to an optimized enterotoxigenic *Escherichia coli-*producing polyvalent antigen gene sequence and application thereof in preventing weaned piglet diarrhea.

Various types of vaccines are also used for diseases associated with weaning piglets, providing passive or active immunity, depending on the type of preparation. Parenteral vaccines for pregnant sows used in Poland are Scanoporc EC/LT/CP, Kolisin Neo, Rokovac Neo, Coliporc, Clostriporc Coli, Gletvax 6. There are also marketed vaccines containing isolated fimbrial adhesins, such as Porcilis ColiClos, Porcilis Porcoli Diluvac Forte, Neocolipor, Suiseng, Enteroporc Coli AC. Vaccination of sows results in high titers of specific antibodies in sow colostrum and milk, which are taken up by piglets through this route giving them temporary passive immunity. An oral Coliprotec F4/F18 vaccine containing live attenuated bacteria should be available on the European market (which is supposed to provide protection against PWD and ED, but the Applicant has not been able to purchase it anywhere), intramuscular subunit vaccines with genetically modified Stx2e toxin protein-Ecoporc Shiga and Vepured (protection against ED), and an intramuscular vaccine with inactivated bacteria and LT toxoid-Scanoporc EC/LT/CP (protection against diarrhea). However, no vaccine can provide 100% immunity. Nonetheless, the time of immune response generation by the piglet is a high-risk moment for infection and infection development.

The aim of the present invention was to develop a new preparation containing inactivated antigens enriched with Stx protein and IgY antibodies, providing enhanced immunity to post-weaning diarrhea (PWD) and edema disease (ED) in piglets, reduced symptoms in case of infection and with positive effect on piglet health.

This aim was reached by the subject matter of the present invention, which is a vaccine for use in the prevention and/or treatment of colibacterioses in piglets, which contains:
- a mixture of four strains of whole inactivated *Escherichia coli* bacteria, strains obtained from animals with symptoms of either post-weaning diarrhea or edema disease, with the following characteristics:

| **Feature/strain** | **A4** | **V5** | **K2** | **K3** |
|---|---|---|---|---|
| Surface antigens (serologically determined) | O:139 | O:149 | O:139, F:4 | O:139 |
| Genes, surface antigens (determined by PCR) | F18, STb | F18, STb, LT, AIDA, F5, and F41 | F4, STb, F5 | Stx2e, STb, F5 |
| Antibiotic resistance | gentamicin, rifampicin, cefalotin | rifampicin, cefalotin | rifampicin, chloramphenicol | rifampicin, chloramphenicol |

and
- Stx2eB proteins with sequence **SEQ ID NO: 1** and/or **SEQ ID NO: 3** and/or **SEQ ID NO: 5.**

Preferably, the vaccine contains additives such as a pharmaceutically or veterinary acceptable carrier such as saline and an adjuvant.

Preferably, the vaccine contains a mixture of four strains of whole inactivated *Escherichia coli* bacteria mixed in a ratio of 1:1:1:1.

Preferably, the vaccine contains:
1-5×10⁹ CFU of a mixture of four strains of whole inactivated *Escherichia coli* bacteria mixed in a ratio of 1:1:1:1
   and
50-500 µg of Stx2eB proteins with the sequence **SEQ ID NO: 1** and/or **SEQ ID NO: 3** and/or **SEQ ID NO: 5.**

Preferably, the vaccine is for administration by injection.

The subject matter of the invention is also a method for obtaining the above-defined vaccine, including steps in which:
- Stx2eB protein is produced;
- a mixture of four strains of whole inactivated *Escherichia coli* bacteria is prepared, followed by
- mixing the produced Stx2eB protein with the mixture of four strains of whole inactivated *Escherichia coli* bacteria;
   wherein
- the Stx2eB protein is produced by culturing an expressive E. coli strain transformed with a gene encoding the Stx2eB protein or its modified version, and
- the mixture of inactivated strains is obtained by inactivating the bacterial culture with 10% formalin.

The invention also relates to a use of the above-defined vaccine for production of an immunizing and/or therapeutic preparation against colibacterioses in piglets.

The subject matter of the invention is also an immunizing and/or therapeutic preparation against colibacterioses in piglets, which comprises:
- the above-defined vaccine for administration by injection, and
- chicken IgY antibodies to E. coli strains having O:139, O:149, F:4, F18, F5 antigens, for oral administration.

Preferably, the preparation contains additives such as a pharmaceutically or veterinary acceptable carrier such as saline, an adjuvant, a preservative such as thimerosal.

Preferably, chicken IgY antibodies to E. coli strains having O:139, O:149, F:4, F18, F5 antigens were obtained by immunizing chickens with four *Escherichia coli* strains with the following characteristics:

| **Feature/strain** | **A4** | **V5** | **K2** | **K3** |
|---|---|---|---|---|
| Surface antigens (serologically determined) | O:139 | O:149 | O:139, F:4 | O:139 |
| Genes, surface antigens (determined by PCR) | F18, STb | F18, STb, LT, AIDA, F5, and F41 | F4, STb, F5 | Stx2e, STb, F5 |
| Antibiotic resistance | gentamicin, rifampicin, cefalotin | rifampicin, cefalotin | rifampicin, chloramphenicol | rifampicin, chloramphenicol |

Preferably, chicken IgY antibodies obtained by immunization of chickens with four strains of *Escherichia coli* with the characteristics defined in claim 10 are combined in a ratio of 1:1:1:1.

Preferably, the preparation contains 1-5×10⁹ CFU of a mixture of four strains of whole inactivated *Escherichia coli* bacteria mixed in a ratio of 1:1:1:1 and 50-500 µg of Stx2eB proteins with the sequence **SEQ ID NO: 1** and/or **SEQ ID NO: 3** and/or **SEQ ID NO: 5** in the form of a vaccine for administration by injection and chicken IgY antibodies to E. coli strains having O:139, O:149, F:4, F18, F5 antigens, for oral administration in the amount of 15-500 mg.

Preferably, the preparation is administered in two doses at an interval of either 7 or 14 days.

Preferably, the preparation is administered according to the following scheme:

| Administration | Age of the animal | Composition of the injection dose | Composition of the oral dose |
|---|---|---|---|
| First dose | The 18th day of life of the piglet | 10⁹ CFU of a mixture of 4 antigens mixed in a ratio of 1:1:1:1, 0.25x10⁹ of each strain and 50 µg of Stx2eB protein | 15 mg of IgY antibodies mixed in a ratio of 1:1:1 :1, 3.75 mg of each type of antibody |
| Second dose | The 32nd day of life of the piglet | 5x10⁹ CFU of a mixture of 4 antigens mixed in a ratio of 1:1:1:1, 1.25x10⁹ of each strain and 100 µg of Stx2eB protein | 15 mg of IgY antibodies mixed in a ratio of 1:1:1 :1, 7.5 mg of each type of antibody |

Preferably, the chicken IgY antibodies are administered daily in an amount of 15-500 mg/day.

The preparation according to the present invention administered bi-directionally protects the piglet similarly to sow's colostrum. The IgY antibodies neutralize pathogenic bacteria that could enter the piglet's digestive system before its immune system responds to the vaccine.

This preparation is composed of two parts: 1) a vaccine for administration by injection, preferably intramuscular, in liquid form, as an injectable solution (inactivated antigens and protein), and 2) a preparation for oral (per os), administration in a liquid form, as a drinking solution (IgY antibodies). The products are designed for piglets of peri-weaning age. The preparations protect against pathogenic strains of E. coli that cause colibacterioses in piglets: **post-weaning diarrhea** (PWD) and **edema disease** (ED), i.e. peri-weaning age diseases. The study showed protection against both of the above disease entities.

### EXAMPLES

### Example 1. Preparation/COLIFOX vaccine

### COMPOSITION:

### I.Active substances administered by injection:

1) A mixture of 4 strains of *Escherichia coli* (whole bacteria) inactivated with formaldehyde. The strains were obtained from animals with symptoms of PWD and ED.
Characteristics of these strains:

| **Feature/strain** | **A4** | **V5** | **K2** | **K3** |
|---|---|---|---|---|
| Surface antigens (serologically determined) | O:139 | O:149 | O:139, F:4 | O:139 |
| Genes, surface antigens (determined by PCR) | F18, STb | F18, STb, LT, AIDA, F5, and F41 | F4, STb, F5 | Stx2e, STb, F5 |
| Antibiotic resistance | gentamicin, rifampicin, cefalotin | rifampicin and cefalotin | rifampicin and chloramphenicol | rifampicin and chloramphenicol |

2) Stx2eB protein-a protein obtained by genetic modification (for overproduction) of a strain by the Stx2e toxin subunit B gene derived from a field strain (gene insertion into a His-tagged plasmid by Gibson method, transformation of an expressive *E. coli* DE3 strain), purified by chromatography on a nickel bed.

### Additional substances:

3) saline, adjuvant, preferably Montanide Gel02 (20%).

Preferably, in addition:

### II. Active substances administered orally:

1) IgY antibodies to *E coli* strains, including A4, V5, K2 and K3, having antigens *i.a*. O:139, O:149, F:4, F18, F5, combined in a ratio of 1:1:1:1 and administered at 15-500 mg/day from the day of administration of the first dose of the vaccine until the 14th day after the second dose of the vaccine (46th day of life of the piglet).

### Additional substances:

2) saline, thimerosal 0.01%.

### Suggested administration scheme:

| Administration | Age of the animal | Composition of the injection dose | Composition of the oral dose |
|---|---|---|---|
| First dose | The 18th day of life of the piglet | 10⁹ CFU of a mixture of 4 antigens (mixed in a ratio of 1:1:1:1, 0.25x10⁹ of each strain) and 50 µg of Stx2eB protein | 15 mg of IgY antibodies (mixed in a ratio of 1:1:1:1 with 3.75 mg of each type of antibody) |
| Second dose | The 32nd day of life of the piglet | 5×10⁹ CFU of a mixture of 4 antigens (mixed in a ratio of 1:1:1:1, 1.25x10⁹ of each strain) and 100 µg of Stx2eB protein | 15 mg of IgY antibodies (mixed in a ratio of 1:1:1:1 with 7.5 mg of each type of antibody) |

In general, the dose range can be defined in the following ranges:
1-5×10⁹ CFU of a mixture of 4 antigens (mixed in a ratio of 1:1:1:1) and 50-500 µg of Stx2eB protein for administration by injection
   AND
15-500 mg of IgY/day for oral administration.

### Stx2eB protein

### Stx2eB sequence

Amino acid sequence: 
ADCAKGKIEFSKYNEDNTFTVKVSGREYWTNRWNLQPLLQSAQLTGMTVTIISNTCSSGSGFAQVK
FN (SEQ ID NO: 1)
Nucleotide sequence:

gcggattgtgctaaaggtaaaattgagttttccaagtataatgaggataatacctttactgtgaaggtgtcaggaagagaatactggacgaacagatggaa

tttgcagccattgttacaaagtgctcagctgacagggatgactgtaacaatcatatctaatacctgcagttcaggctcaggctttgcccaggtgaagttta

actga (SEQ ID NO: 2)

### Stx2eB sequence with His-tag

C-HIS variant amino acid sequence: 
HHHHHHADCAKGKIEFSKYNEDNTFTVKVSGREYWTNRWNLQPLLQSAQLTGMTVTIISNTCSSG
SGFAQVKFN (SEQ ID NO: 3)
C-HIS variant nucleotide sequence: 
ATGCACCACCATCATCACCACgcggattgtgctaaaggtaaaattgagttttccaagtataatgaggataatacctttactgtgaagg
tgtcaggaagagaatactggacgaacagatggaatttgcagccattgttacaaagtgctcagctgacagggatgactgtaacaatcatatctaatacctgc
agttcaggctcaggctttgcccaggtgaagtttaactga (SEQ ID NO: 4)
N-HIS variant amino acid sequence: 
MADCAKGKIEFSKYNEDNTFTVKVSGREYWTNRWNLQPLLQSAQLTGMTVTIISNTCSSGSGFAQ
VKFNHHHHHH (SEQ ID NO: 5)
N-HIS variant nucleotide sequence:

ATGgcggattgtgctaaaggtaaaattgagttttccaagtataatgaggataatacctttactgtgaaggtgtcaggaagagaatactggacgaacaga
tggaatttgcagccattgttacaaagtgctcagctgacagggatgactgtaacaatcatatctaatacctgcagttcaggctcaggctttgcccaggtgaa
gtttaacCACCACCATCATCACCACtga (SEQ ID NO: 6)

### Example of how to obtain this protein

### Procedure

Rejuvenate the overnight bacterial culture (expressive *E coli* strain with the Stx2eB protein encoding gene or a modified version thereof) in a sterile medium with the addition of suitable selection markers (e.g., an antibiotic) and cultivate the new culture at 20 to 37 °C until a suitable optical density is achieved, after which induce with a suitable inducer (e.g., IPTG) and continue the culture at 20 to 37 °C for 3 to 24 hours. After the culture is completed, centrifuge and discard the supernatant. Suspend the cell pellet in a suitable buffer and sonicate, then centrifuge. Purify the filtered supernatant by gradient chromatography in successive buffers differing in the composition/concentration of the eluting component/pH. Ultra/diafilter the filtration fraction into the preparation buffer until a suitable concentration is reached.

### Inactivated strain mixture

### Brief description of how to obtain a mixture of these inactivated strains

Inoculate LB medium with overnight bacterial culture at a ratio of 1:200. Cultivate the culture at 37 °C with aeration for 24 h. Optionally, an antifoaming agent may be added. Inactivate the culture with 10% formalin to obtain a concentration of 0.2% and incubate further at 37 °C with agitation without addition of air for at least 3 h. Then transfer the culture to a sterile dish, seal tightly and inactivate further overnight under the same conditions. Centrifuge the inactivated bacteria and suspend the pellet in 0.85% NaCl with 0.02% formalin.

### Possible adjuvants

Exemplary adjuvants not limited to: Montanide Gel02 (20%), Incomplete Freund's Adjuvant (IFA), MONTANIDE ISA 25, Aluminum hydroxide and others.

### Example 2. Interaction of isolated IgYs with live bacteria in liquid medium

The aim of the study was to investigate which IgY concentration has an inhibitory effect on the growth of *E coli* bacteria. The study used produced and isolated IgY antibodies. After the antibodies were isolated, tube agglutination was performed to determine the titer of specific antibodies. An ELISA assay was also performed to determine the concentration of specific antibodies relative to a standard curve for standard IgYs of known concentration.

| IgY series | The antigen with which the chickens were vaccinated | Titer determined by tube agglutination |
|---|---|---|
| EC/001/21 | A4 | 1/160 |
| EC/002/21 | A4 | 1/80 |
| EC/003/21 | V5 | 1/160 |

Tube agglutination of antibodies was performed with all four antigens used for vaccinations-there were minor co-agglutinations (up to dil. 1/20), indicating that the strains used differed in surface antigens.

### 1. DESCRIPTION OF THE EXPERIMENTS

### First trial

a. 50 mL of LB was inoculated with *E coli* A4 and V5 strains for revival. The cultures were incubated overnight at 37 °C with 150 rpm shaking.
b. From overnight cultures of A4 and V5 strains, a suspension with turbidity of 2 McF (6×10⁸ CFU/mL) was prepared in sterile tubes.
c. 20 flasks were prepared with 27 mL of LB broth, 3 mL of the prepared A4 strain suspension was added to 10 flasks and 3 mL of V5 strain suspension was added to the remaining ones and a starting bacterial count of 6×10⁷ CFU/mL was obtained.
d. 3 dilutions of IgY antibodies were prepared from the starting concentration of 4x (concentration vs. starting volume of yolk): 1:5, 1:20 and 1:50.
e. Before incubation, IgY EC/001 (anti-A4) antibodies were added to 3 flasks of each strain in amounts of: 1 mL of 1:5 IgY, 1 mL of 1:20 and 1 mL of 1:50, and one flask was labeled K(-) and no antibodies were added to it throughout the study.
f. Turbidity was measured at time 0 and all flasks were placed in an incubator a 37 °C with 150 rpm shaking.
g. After 3 h of culture, the OD of all samples was measured and IgY antibodies or salt were added to the next 3 flasks as in c and the flasks were again placed in the incubator.
h. After 6 h, the OD measurement was repeated and IgY antibodies or salt were added to the last 3 flasks.
i. Cultures were incubated overnight at 37 °C with 150 rpm shaking.
j. After 24 h, the OD was measured again in all culture dishes after which the cultures were disposed of in an autoclave.

### Second trial

Because the bacteria grew too fast (after 3 h the turbidity was about 3 McF), it was decided to repeat the test using a lower amount of bacteria in the starting culture-in the first trial it was started at 10⁷ CFU/mL, in the second trial the starting amount of bacteria was changed to 10⁵ CFU/mL, the test was performed only for A4 strain and the addition of antibodies was tested without dilution (1x).

### Third trial

At the second trial it was noted that for about 6 h after the addition of IgY, the bacteriostatic effect persisted and at the next measurement the bacteria began to grow again. For this reason, it was decided to conduct an additional test, in which it was decided to test the multiple additions of IgY antibodies, wherein different doses of IgY were tested in re-administration.

### How the experiment was conducted (third trial)

| Culture duration | K(-) | 1 | 2 | 3 | 4 | 5 | 6 | 7 |
|---|---|---|---|---|---|---|---|---|
| 0 h | | 1x IgY addition | 1x IgY addition | 1x IgY addition | 1x IgY addition | 1x IgY addition | 1x IgY addition | 1x IgY addition |
| 7h | | - | 1x IgY addition | 1:5 IgY addition | 1:10 IgY addition | 1x IgY addition | 1:5 IgY addition | 1:10 IgY addition |
| 24 h | | - | - | - | - | 1x IgY addition | 1:5 IgY addition | 1:10 IgY addition |

| Culture duration | 8 | 9 | 10 | 11 | 12 | | | |
|---|---|---|---|---|---|---|---|---|
| 0h | 1:5 IgY addition | 1:5 IgY addition | 1:5 IgY addition | 1:5 IgY addition | 1:10 IgY addition | | | |
| 7 h | - | 1:5 IgY addition | 1:5 IgY addition | 1:10 IgY addition | 1:10 IgY addition | | | |
| 24 h | - | - | 1:5 IgY addition | 1:10 IgY addition | 1:10 IgY addition | | | |

### 2. RESULTS

### Measurement results (first trial)-starting bacterial count: 10⁷ CFU/mL

| A4 strain + IgY anti-A4 | | 0 h | 3h | 6 h | 24 h |
|---|---|---|---|---|---|
| K(-) | - | 0.2 | 3.1 | 3.9 | 6.89 |
| IgY 1:5 | IgY addition | 0.2 | 2.19 | 3.8 | 5.2 |
| IgY 1:20 | at 0 h | 0.2 | 2.37 | 3.72 | 5.13 |
| IgY 1:50 | | 0.2 | 2.79 | 2.84 | 5.28 |
| IgY 1:5 | IgY addition at 3 h | 0.2 | 3.0 | 3.53 | 4.95 |
| IgY 1:20 | | 0.2 | 3.01 | 2.56 | 5.21 |
| IgY 1:50 | | 0.2 | 2.95 | 2.78 | 5.06 |
| IgY 1:5 | IgY addition at 6 h | 0.2 | 3.05 | 3.5 | 5.03 |
| IgY 1:20 | | 0.2 | 3.0 | 3.6 | 4.78 |
| IgY 1:50 | | 0.2 | 2.98 | 3.56 | 5.25 |

| V5 strain + IgY anti-A4 | | 0 h | 3h | 6 h | 24 h |
|---|---|---|---|---|---|
| K(-) | - | 0.2 | 3.34 | 3.82 | 7.67 |
| IgY 1:5 | IgY addition at 0 h | 0.2 | 3.17 | 3.82 | 7.29 |
| IgY 1:20 | | 0.2 | 3.22 | 3.98 | 6.92 |
| IgY 1:50 | | 0.2 | 3.22 | 3.78 | 6.55 |
| IgY 1:5 | IgY addition at 3 h | 0.2 | 3.22 | 3.84 | 7.33 |
| IgY 1:20 | | 0.2 | 3.29 | 3.91 | 6.98 |
| IgY 1:50 | | 0.2 | 3.16 | 4.05 | 7.4 |
| IgY 1:5 | IgY addition at 6 h | 0.2 | 3.19 | 3.78 | 6.91 |
| IgY 1:20 | | 0.2 | 3.24 | 4.05 | 7.12 |
| IgY 1:50 | | 0.2 | 3.31 | 3.8 | 6.39 |

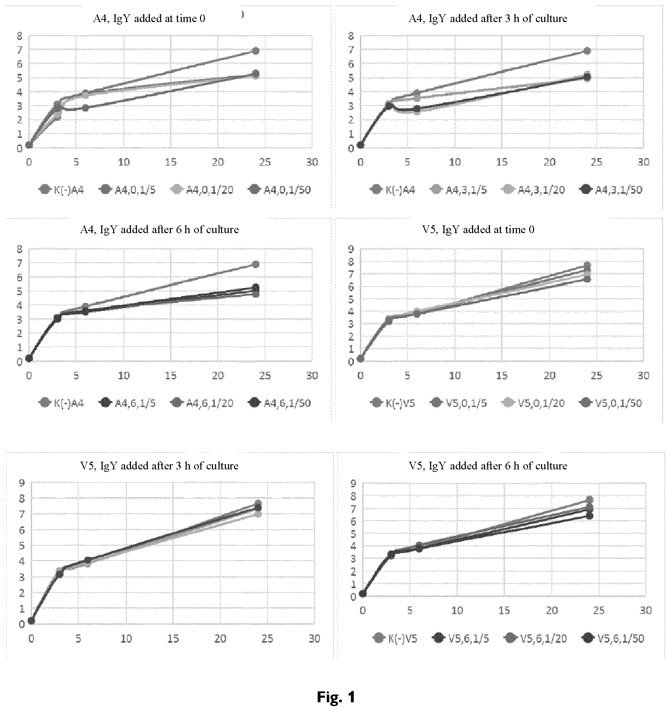

Strain growth charts (first trial) are shown in Fig. 1. V5 strain growth was not inhibited at all with anti-A4 antibodies. In case of A4 strain due to too rapid growth of bacteria (too large inoculum), the desired effect could not be observed-in flasks containing 1:5 and 1:20 antibodies, a clear deposit of agglutinates on the bottom of the flask was observed.

### Measurement results (second trial)-starting bacterial count: 10⁵ CFU/mL

| A4 strain + IgY anti-A4 | | 0 h | 3 h | 6 h | 24 h |
|---|---|---|---|---|---|
| K(-) | - | 0.1 | 0.51 | 3.2 | 6.48 |
| IgY 1x | IgY addition at 0 h | 0.1 | 0.16 | 0.2 | 2.38 |
| IgY 1:5 | | 0.1 | 0.11 | 0.46 | 4.38 |
| IgY 1:20 | | 0.1 | 0.32 | 0.85 | 5.35 |
| IgY 1:50 | | 0.1 | 0.31 | 1.29 | 5.53 |
| IgY 1x | IgY addition at 3 h | 0.1 | 0.56 | 0.59 | 1.89 |
| IgY 1:5 | | 0.1 | 0.65 | 0.65 | 4.13 |
| IgY 1:20 | | 0.1 | 0.73 | 0.78 | 4.98 |
| IgY 1:50 | | 0.1 | 0.47 | 0.99 | 5.47 |
| IgY 1x | IgY addition at 6 h | 0.1 | 0.52 | 3.05 | 2.6 |
| IgY 1:5 | | 0.1 | 0.46 | 3.02 | 3.93 |
| IgY 1:20 | | 0.1 | 0.62 | 3.06 | 4.38 |
| IgY 1:50 | | 0.1 | 0.55 | 2.9 | 4.75 |

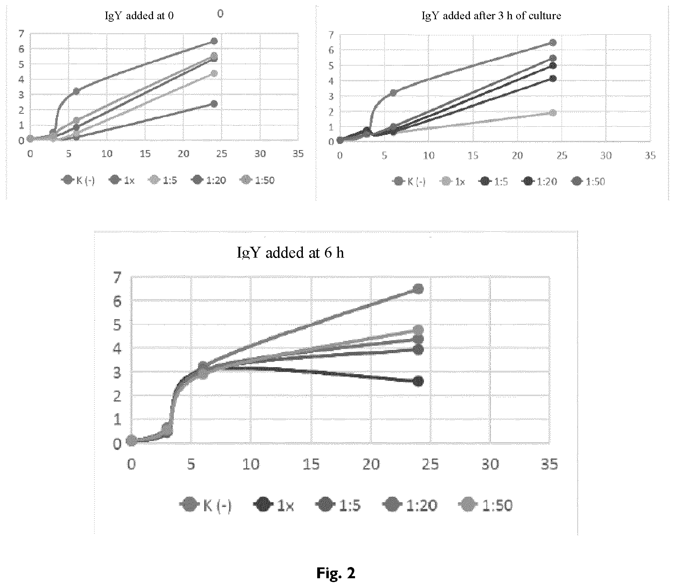

Strain growth charts of the second trial are shown in Fig. 2. At the second trial, a strong bacteriostatic effect was observed up to about 6 h after the addition of antibodies at each time point with a dilution of 1x, 1:5, and 1:20, at 1:50 dilution the bacteria grew similarly to the control culture.

### Measurement results (third trial)

| Samples | | 0 h | 3,5 h | 7 h | 24 h | 30 h | 48 h |
|---|---|---|---|---|---|---|---|
| K(-) | | 0.05 | 0.89 | 3.11 | 6.55 | 6.71 | 6.67 |
| 1 | 1x conc. IgY at 0 h | 0.05 | 0.05 | 0.18 | 3.37 | 3.95 | 5.17 |
| 2 | 1x conc. IgY at 0 h and 7 h | 0.05 | 0.08 | 0.07 | 2.52 | 3.8 | 5.89 |
| 3 | 1x conc. IgY at 0 h and 1:5 conc. at 7 h | 0.05 | 0.01 | 0.05 | 2.08 | 2.98 | 4.14 |
| 4 | 1x conc. IgY at 0 h and 1:10 conc. at 7 h | 0.05 | 0.07 | 0.07 | 2.84 | 3.64 | 4.33 |
| 5 | 1x conc. IgY at 0 h, 7 h, and 24 h | 0.05 | 0.03 | 0.04 | 2.1 | 2.52 | 3.98 |
| 6 | 1x conc. IgY at 0 h and 1:5 conc. at 7 h, and 24h | 0.05 | 0.12 | 0.07 | 1.92 | 2.79 | 3.88 |
| 7 | 1x conc. IgY at 0 h, 1:10 conc. at 7 h, and 24 h | 0.05 | 0.07 | 0.08 | 2.17 | 2.86 | 3.97 |
| 8 | 1:5 conc. IgY at 0 h | 0.05 | 0.17 | 0.54 | 4.51 | 4.8 | 5.91 |
| 9 | 1:5 conc. IgY at 0 h and 7 h | 0.05 | 0.23 | 0.52 | 3.95 | 4.59 | 4.2 |
| 10 | 1:5 conc. IgY at 0 h, 7 h, and 24 h | 0.05 | 0.22 | 0.58 | 3.75 | 3.9 | 4.29 |
| 11 | 1:5 conc. IgY at 0 h, 1:10 conc. at | 0.05 | 0.26 | 0.53 | 3.93 | 3.53 | 3.81 |
| | 7 h, and 24 h | | | | | | |
| 12 | 1:10 conc. IgY at 0 h, 7 h, and 24 h | 0.05 | 0.25 | 0.5 | 4.06 | 3.64 | 2.98 |

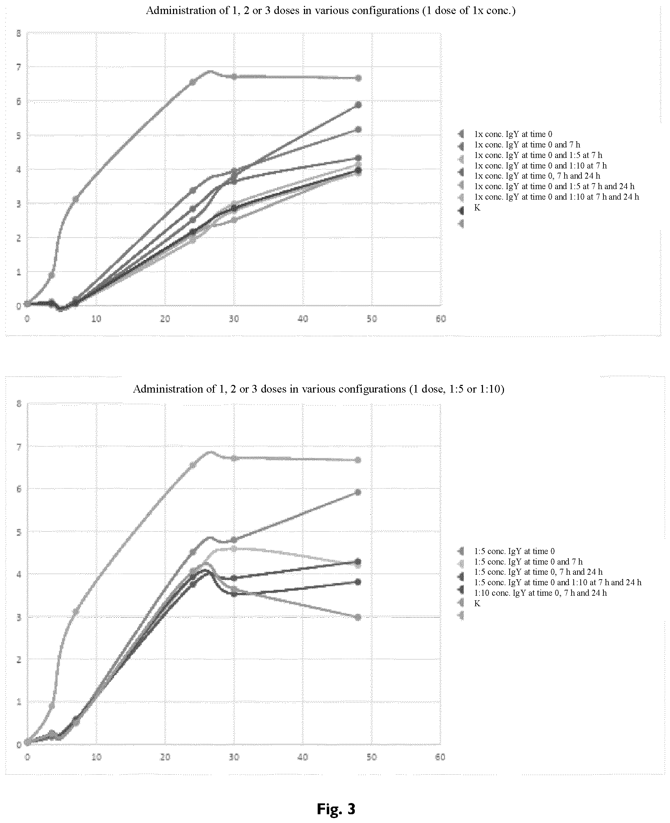

Strain growth charts of the third trial are shown in Fig. 3. The results are for samples in which a dilution of 1x IgY was used as the first "dose" administered at time 0. Subsequent doses of IgY in different configurations were added at 7 h and 24 h of culture (see legend description). Triple administration of antibodies, regardless of concentration, resulted in the greatest reduction in growth-the best bacteriostatic effect.

**CONCLUSIONS:** In the first trial, a clear agglutination of bacteria was observed when A4 strain was tested with anti-A4 antibodies. In each flask, there was a very large amount of agglutinates at the bottom, but nevertheless further bacterial growth was observed. The final turbidity was about 1 McF lower than in the control to which no antibodies were added, and a mild bacteriostatic effect was observed. In a test V5 strain with anti-A4 antibodies, no growth inhibitory effect or apparent agglutination was observed (in tube agglutination, this strain reacted with antibodies to a dilution of 1:20).

In the second trial, a correspondingly smaller inoculum (10⁵ CFU/mL) was used and it was possible to observe a bacteriostatic effect for about 6 h after the addition of IgY regardless of whether antibodies were added at time of 0 h, 3 h or 6 h after the start of culture, but when measured 24 h after the start of culture, the growth was significant. Application of 1x conc. of IgY resulted in a significant attenuation of growth compared to the control (K: 6.48 McF, samples tested: time 0 h: 2.38 Mc, time 3 h: 1.89 McF, time 6: 2.6 McF).

### Example 3: Rat studies

### First round

The aim of the study was to administer two components of COLIFOX vaccine (antigen mixture or Stx2eB protein) to animals in different variants and choose the most optimal variant to administer in the final vaccine composition. In the first round of the study, 5 study groups were used to administer different antigen variants (A1, A2, B1, B2, C1) and 8 study groups to administer different protein variants (D1, D2, D3, E1, E2, E3, F1, F2), the control group was common for both.

### Distribution of groups and preparation administration scheme:

| | Day 0 | Day 7 | Day 14 | Animal number |
|---|---|---|---|---|
| A1 | 5x10⁸ CFU | 5x10⁸ CFU | - | 10 |
| B1 | 5x10⁸ CFU | - | 5x10⁸ CFU | 10 |
| A2 | 5x10⁸ CFU | 10⁹ CFU | - | 10 |
| B2 | 5x10⁸ CFU | - | 10⁹ CFU | 10 |
| C1 | 5x10⁸ CFU | - | - | 12 |
| K(-) | 0.85% NaCl + adjuvant | 0.85% NaCl + adjuvant | 0.85% NaCl + adjuvant | 12 |
| D1 | 10 µg Stx2eB | 10 µg Stx2eB | - | 10 |
| E1 | 10 µg Stx2eB | - | 10 µg Stx2eB | 10 |
| F1 | 10 µg Stx2eB | - | - | 12 |
| D2 | 20 µg Stx2eB | 20 µg Stx2eB | - | 10 |
| E2 | 20 µg Stx2eB | - | 20 µg Stx2eB | 10 |
| F2 | 20 µg Stx2eB | - | - | 12 |
| D3 | 10 µg Stx2eB | 20 µg Stx2eB | - | 10 |
| E3 | 10 µg Stx2eB | - | 20 µg Stx2eB | 10 |

The antigen mixture contained 4 *E coli* strains: A4, V5, K2 and K3 mixed in a ratio of 1:1:1:1.

### DESCRIPTION OF THE EXPERIMENTS

a. Animal injection mixtures were prepared (0.1 mL per dose) and cultures were performed for sterility check.
b. The study was started, during which samples of blood collected from rats (on days 0, 7, 14, 21, 28, 35, and 42) and intestinal sections (from days 14 and 42) were sent to the laboratory.
c. Blood was centrifuged immediately upon obtaining at 8000 rpm, 3 min, serum was transferred to new tubes and stored at -20 °C until analysis.
d. A series of ELISA assays were performed to determine the level of specific IgG antibodies in the sera (directed against the antigen and against Stx2eB). The assay used standard IgG antibodies of known concentration to plot a curve and calculate the antibody concentration [µg/mL]. The assay was performed in a standard way, using a plate washer (3 washes between each step), anti-IgG*HRP conjugate was used at a dilution of 1:40,000.
e. IgA antibody isolation was performed from intestinal sections by grinding each sample (0.5-1 cm intestinal sections were obtained) and re-suspending it in 0.3 mL of saponin buffer (2% saponin, PIC, 0.1% BSA in PBS). The samples were incubated overnight at 4 °C on a rocking shaker, and the next day centrifuged twice with transferring the supernatant to new tubes.
f. From the prepared suspensions, the levels of IgA antibodies to both antigen and Stx2eB were also analyzed. A standard curve was prepared and the conjugate was used at a dilution of 1:50,000.
g. The means and concentrations in each sample were calculated, then the mean levels for each group were calculated and a chart of IgG and IgA level changes during the study was prepared.

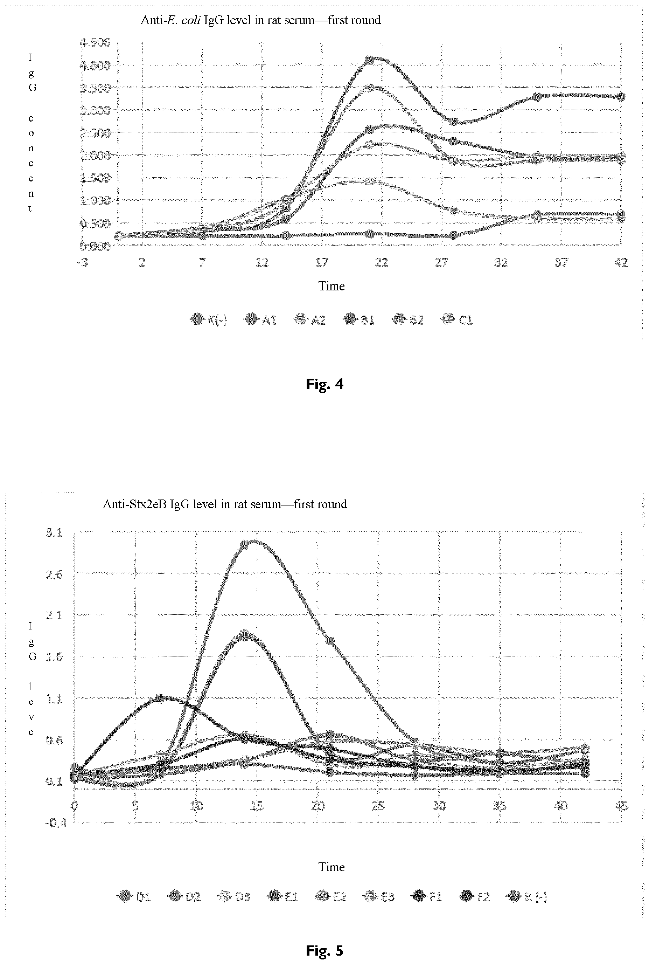

A chart showing the level of anti-antigen IgG is shown in Fig. 4. The highest level was obtained for group B1 vaccinated on day 0 with a dose of 5×10⁸ CFU and then on day 14 with 10⁹ CFU-about 3.3 µg/mL of specific IgGs. The weakest result was obtained by vaccination with a single dose (group C1), by day 42 the IgG level dropped to that of the control group. The other groups (A1, A2, B2) also showed an increase in the level of specific antibodies-about 2 µg/mL.

A chart showing the level of anti-Stx2eB protein IgG is shown in Fig. 5.

When the rats were immunized with various doses of the protein, the results obtained were lower than for the antigen, in addition, the level of antibodies at the end of the study in all groups dropped to baseline. The best effects were observed for the D1 group immunized on days 0 and 7 with a lower dose of protein (10 µg), the higher doses gave the weakest effect and for this reason, versions of an even lower protein dose administration were tested in the next round.

### Anti-antigen IgA level

There was no increase in antibody levels compared to the control group.

### Anti-protein IgA level

There was no increase in antibody levels compared to the control group.

### CONCLUSIONS

In the second round of the study, the preparation will be administered on days 0 and 14 (this version gave the best results for IgG levels for antigen). There will be 3 groups of 10 individuals tested, in which the antigen will be administered as established in the first round (first dose: 5×10⁸ CFU and second dose: 10⁹ CFU) and the protein will be administered in three versions:
1) first dose: 5 µg, second dose: 5 µg
2) first dose: 10 µg, second dose: 10 µg
3) first dose: 5 µg, second dose: 10 µg

### Second round

The purpose of the experiments was to administer a preparation consisting of two COLIFOX vaccine components (antigen mixture or Stx2eB protein) to animals in different variants and choose the most optimal variant to administer in the final vaccine composition. In the second round of the study, 5 study groups were used to administer different antigen variants (A1, A2, B1, B2, C1) and 8 study groups to administer different protein variants (D1, D2, D3, E1, E2, E3, F1, F2), the control group was common for both.

Distribution of groups and preparation administration scheme:

| | Day 0 | Day 14 | Animal number |
|---|---|---|---|
| G | 5x10⁸ CFU + 5 µg of the protein | 10⁹ CFU + 5 µg of the protein | 10 |
| H | 5x10⁸ CFU + 10 µg of the protein | 10⁹ CFU + 10 µg of the protein | 10 |
| I | 5x10⁸ CFU + 5 µg of the protein | 10⁹ CFU + 10 µg of the protein | 10 |
| K(-) | 0.85% NaCl + Gel02 | 0.85% NaCl + Gel02 | 12 |

The antigen mixture contained 4 E coli strains: A4, V5, K2 and K3 mixed in a ratio of 1:1:1:1.

### DESCRIPTION OF THE EXPERIMENTS

a. Animal injection mixtures were prepared (0.1 mL per dose) and cultures were performed for sterility check (all preparations were sterile).
b. The study was started, during which samples of blood collected from rats (on days 0, 7, 14, 21, 28, 35, and 42) and intestinal sections (from days 0 and 42) were sent to the laboratory.
c. Blood was centrifuged immediately upon obtaining at 8000 rpm, 3 min, serum was transferred to new tubes and stored at -20 °C until analysis.
d. A series of ELISA assays were performed to determine the level of specific IgG antibodies in the sera (directed against the antigen and against Stx2eB). The assay used standard IgG antibodies of known concentration to plot a curve and calculate the antibody concentration [µg/mL]. The assay was performed in a standard way, using a plate washer (3 washes between each step), anti-IgG*HRP conjugate was used at a dilution of 1:40,000.
e. IgA antibody isolation was performed from intestinal sections by grinding each sample (0.5-1 cm intestinal sections were obtained) and re-suspending it in 0.17 mL of saponin buffer (2% saponin, PIC, 0.1% BSA in PBS). The samples were incubated overnight at 4 °C on a rocking shaker, and the next day centrifuged twice with transferring the supernatant to new tubes.
f. From the prepared suspensions, the levels of IgA antibodies to both antigen and Stx2eB were also analyzed. A standard curve was prepared, and the conjugate was used at a dilution of 1:50,000.
g. The means and concentrations in each sample were calculated, then the mean levels for each group were calculated and a chart of IgG and IgA level changes during the study was prepared. Results were as follows.

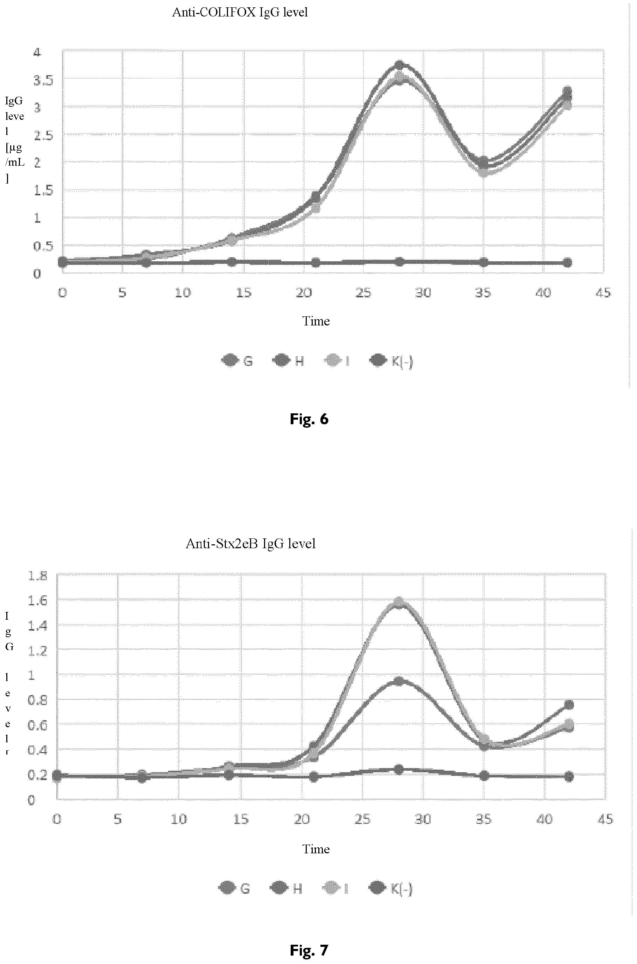

A chart showing the level of anti-antigen IgG is shown in Fig. 6. All groups were vaccinated with the same dose of antigen, therefore the antibody levels are similar, only the protein doses in the preparations differed. The IgG level at the end of the study (day 42) was about 3.1 µg/mL (compared to 3.2 µg/mL in the first round on day 42). The results of anti-COLIFOX IgG levels from the first and second rounds are consistent.

A chart showing the level of anti-Stx2eB protein IgG is shown in Fig. 7.

The highest final level of anti-Stx2eB antibodies was obtained for group H immunized with two doses of 10 µg. The level of IgG is highest around day 28 and then drops sharply (from 1.6 µg/mL to 0.8 µg/mL), the same situation occurred in the first round (then the level dropped from 2.9 µg/mL to 0.3 µg/mL).

### Anti-antigen IgA level

There was no increase in antibody levels compared to the control group.

### Anti-protein IgA level

There was no increase in antibody levels compared to the control group.

### CONCLUSIONS

Administration of both components simultaneously does not change the dynamics of the antibody level increase. The components do not have a negative effect on each other; the level of anti-Stx2e IgG is higher in the second round, it is possible that the administration of Stx2e with antigen "propelled" the acquisition of immunity to Stx2e and therefore the level at the end of the study is slightly higher than in the first round, when the protein was administered without antigen.

### Third round

In the third round of the study, the preparation was administered on days 0 and 14. Twelve study groups of 10 individuals each and 6 control groups of 5 individuals each were studied. The antigen was administered as determined in the first round (first dose of 5×10⁸ CFU and second dose of 10⁹ CFU) and the protein will be administered based on the results of the second round (first dose: 10 µg, second dose: 10 µg). In the third round with each injection dose containing antigen and protein, IgY antibodies were also administered per os at a dose of 0.5 mg per rat.

### Summary of the rat study

- Based on the rat studies **(first round),** the dose of antigen used for vaccination was selected: the best results (amount of anti-antigen IgG) were obtained in vaccination carried out twice, on days 0 and 14 (first dose of 5×10⁸ CFU and second dose of 10⁹ CFU). In each vaccinated group, regardless of the regimen and dose, an increase in body weight was noted compared to the control group. The highest weight gain (about 31.5 g) was observed for group A1 (administration of antigen mixture, dose of 5×10⁸ CFU on days 0 and 7), the lowest weight gain (about 4 g) was obtained by single administration (day 0) of Stx2eB protein at a dose of 20 µg of Stx2eB.
- **Second round** of the study assumed the selection of the most optimal dosing regimen and composition of the vaccine in terms of Stx2eB protein (amount of anti-protein IgG). The doses chosen were: antigen: first dose of 5×10⁸ CFU and second dose of 10⁹ CFU; Stx2eB protein: first dose of 10 µg, second dose of 10 µg. The control group showed the highest weight gain throughout the study: an average of 85.2 g. Rats given Stx2eB protein at various doses with first antigen dose of 5×10⁸ CFU and second antigen dose of 10⁹ CFU showed a significant inhibition of body weight gain (by more than 15 g). Based on the results, it can be concluded that the higher the dose of Stx2eB protein administered, the lower the weight gain.
- Conclusions from the **third round** of the study: The study used 3 control groups of rats for each disease entity, with an average weight gain of about 60.4 g **(PWD group)** and 54.46 g **(ED group).** The vaccine was administered on days 0 and 14 in different ways. Infection occurred on days 7, 14 or 28. The weakest weight gain for PWD averaged 56.4 g in the group where infection was performed on day 7 and administered: first dose of 5×10⁸ CFU i.m. and 0.5 mg of IgY per os, second dose of 10⁹ CFU i.m. and 0.5 mg of IgY *per os,* in the group in which the infection was performed on day 28 (the same dosage and administration of the vaccine). **In the ED group,** a weight gain was noted in all animals compared to the control group (54.4 g), the best results were obtained for the group in which the infection was performed on day 14 and administered according to the scheme: first dose: 5×10⁸ CFU + 10 µg of protein i.m. and 0.5 mg IgY *per os,* second dose 10⁹ CFU + 10 µg of protein i.m. and 0.5 mg IgY *per os* (mean 77.5 g weight gain), the worst result was observed in the group infected on day 28 (same dosage).

### Example 4: Piglet studies

The aim of the study was to test the effectiveness and safety of the preparation according to the present invention against colibacterioses caused by *E coli* strains in piglets. The feed given to piglets was zinc oxide-free.

A test infection was performed-two piglets were administered the strain causing PWD, the other two were administered the strain causing ED. Within 48 h after the 10⁹ CFU oral administration, all 4 piglets developed diarrhea and disease symptoms.

The remaining 36 piglets were randomly divided into 6 groups of 6 as described in the table below. The animals were administered preparations (antigen and/or protein injection, antibodies orally) on days 1 and 14 of the study (18th and 32nd day of life), and were infected with a dose of 10⁹ CFU on day 28 of the study. During the study, samples were collected-rectal swabs and blood samples for further examination, and on the last day of the study, small intestine sections were collected from all animals. During the study, the animals were weighed and the animal body temperature was measured.

The next day CHROMagar ECC medium (selective medium for fecal bacteria growth) and CHROMagar STEC medium (selective medium for Shiga toxin-producing bacteria) were inoculated with swabs to determine the total shedding.

The following were measured from the blood samples: (i) the level of antigen-specific antibodies to the antigen and protein used (IgG in serum, IgA in intestinal sections), (ii) the level of cytokines.

| Group designation | Description | Infection | First dose | Second dose |
|---|---|---|---|---|
| A | Control (K) | ED (F18 + Stx2e) | Salt + adjuvant | Salt + adjuvant |
| B | Control (K) | PWD (F4) | Salt + adjuvant | Salt + adjuvant |
| C | Antigen only (A) | ED (F18 + Stx2e) | 10⁹ CFU of antigen i.m. + 15 mg of IgY | 5x10⁹ CFU of antigen i.m. + 30 |
| | | | *per os* | mg of IgY *per os* |
| D | Antigen only (A) | PWD (F4) | 10⁹ CFU of antigen i.m. + 15 mg of IgY *per os* | 5x10⁹ CFU of antigen i.m. + 30 mg of IgY *per os* |
| E | Antigen + Stx (B) | ED (F18 + Stx2e) | 10⁹ CFU antigen + 50 µg Stx2eB i.m. and 15 mg IgY *per os* | 5x10⁹ CFU of antigen + 75 µg Stx2eB i.m. and 30 mg IgY *per os* |
| F | Antigen + Stx (B) | PWD (F4) | 10⁹ CFU antigen + 50 µg Stx2eB i.m. and 15 mg IgY *per os* | 5x10⁹ CFU antigen + 75 µg Stx2eB i.m. and 30 mg IgY *per os* |

During the study, analysis of colonies growing on the plates was conducted to confirm the presence of the strains with which the animals were infected. Genetic material was isolated from individual colonies or a mixture and then a PCR reaction was carried on for Stx2e, F4 or F18 genes-the presence of genes from the strains used for infection was confirmed in all study groups.

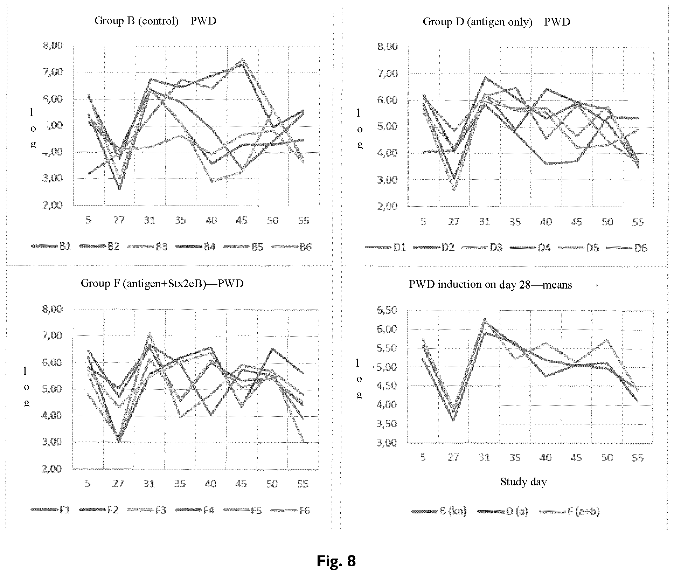

Charts presenting cultures in the PWD-induced groups on day 28 are shown in Fig. 8.

There were no significant differences in shedding between the control and study groups throughout the study period, however, diarrhea was more severe in the control group than in the study groups, but this did not translate into differences in total bacterial counts in swabs from the animals.

There was an increase in the bacterial count in cultures from all groups after infection (day 28), the amounts of bacteria in the swabs from that day onward successively decreased until the end of the study.

With each dose of the preparation (days 0 and 14), the animals also received a mixture of anti*-E coli* IgY antibodies as passive immunization-there was a significant decrease in coliform bacteria numbers in swabs on these days, but it cannot be clearly determined whether it was the effect of the antibodies administered, as the control group (which did not receive antibodies) also showed a decrease in the number of bacteria in swabs.

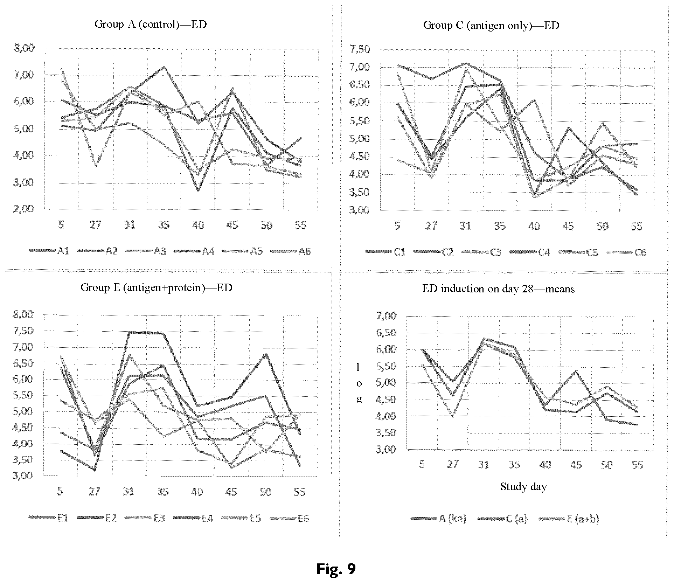

Charts presenting cultures in the ED-induced groups on day 28 are shown in Fig. 9.

As with PWD infection, there were no significant differences in shedding between the control and study groups (only on day 45 there were significantly higher counts in the control group, while on days 50 and 55 the control group had lower counts than the study groups). In case of ED-induced groups, the control group and the antigen only-vaccinated group also had more severe and longer-lasting diarrhea. The antigen only-vaccinated group was not vaccinated with the Stx2e protein and the animals did not have immunity to the strain used for infection, which may indicate that the addition of the protein in the E group contributes to faster elimination of the disease symptoms. These groups also showed an increase in the bacterial count in the cultures after infection (day 28), the amounts of bacteria in the swabs from that day onward successively decreased until the end of the study.

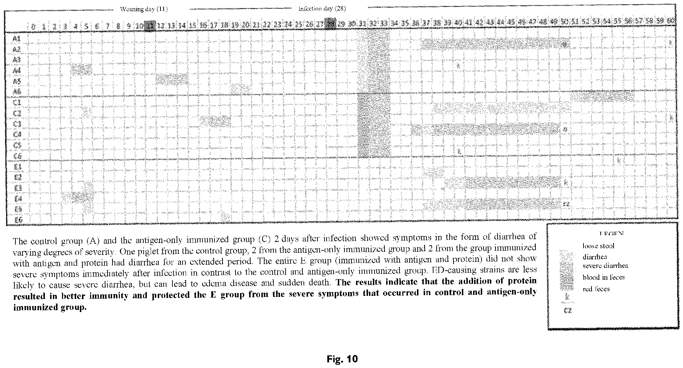

The symptoms observed in piglets with induced ED (F18 + Stx2eB infection) are shown in Fig. 10. The control group (A) and the antigen only-immunized group (C) 2 days after infection showed symptoms in the form of diarrhea of varying degrees of severity. One piglet from the control group, 2 from the antigen only-immunized group and 2 from the group immunized with antigen and protein had diarrhea for an extended period. The entire E group (immunized with antigen and protein) did not show severe symptoms immediately after infection in contrast to the control and antigen only-immunized group. ED-causing strains are less likely to cause severe diarrhea, but can lead to edema disease and sudden death. The results indicate that the addition of protein resulted in better immunity and protected the E group from the severe symptoms that occurred in control and antigen only-immunized group.

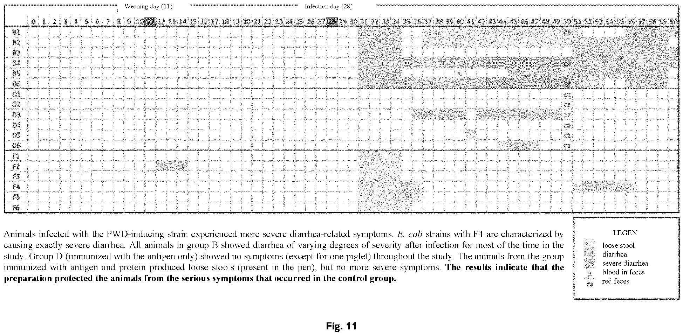

The symptoms observed in piglets with induced PWD (F4 infection) are shown in Fig. 11. Animals infected with the PWD-inducing strain experienced more severe diarrhea-related symptoms. E coli strains with F4 are characterized exactly by causing severe diarrhea. All animals in group B showed diarrhea of varying degrees of severity after infection for most of the time in the study. Group D (immunized with the antigen only) showed no symptoms (except for one piglet) throughout the study. The animals from the group immunized with antigen and protein produced loose stools (present in the pen), but no more severe symptoms. The results indicate that the preparation protected the animals from the serious symptoms that occurred in the control group.

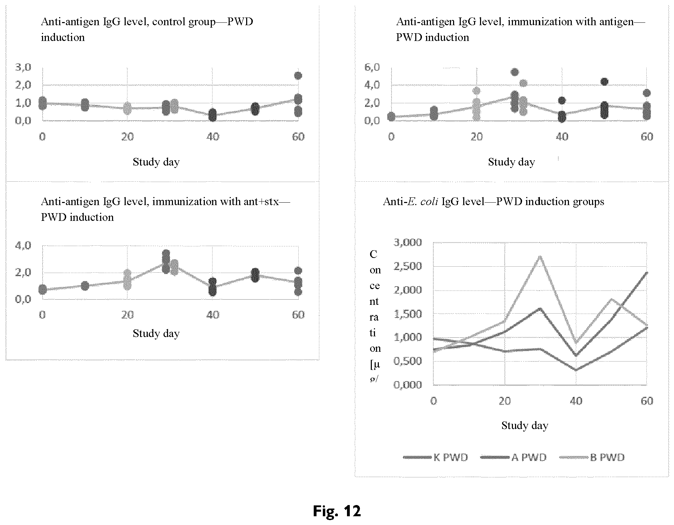

Fig. 12 shows the anti-antigen IgG levels in the PWD-induced groups on day 28.

For all IgG studies, the first three charts show the scatter of results within each group, while the last chart shows the mean measurements for all groups.

Significant increases in the antigen-specific antibodies were shown for the group immunized with antigen and protein and with antigen only. On days 20 to 50, the highest level presented the group immunized with antigen and protein; at the end of the study, the group immunized with antigen only showed a higher level.

At the end of the study, the control group also showed an increase in antibodies, which was due to infection and the body's response to the pathogen administered to cause the disease symptoms, but the levels were still higher in the study groups.

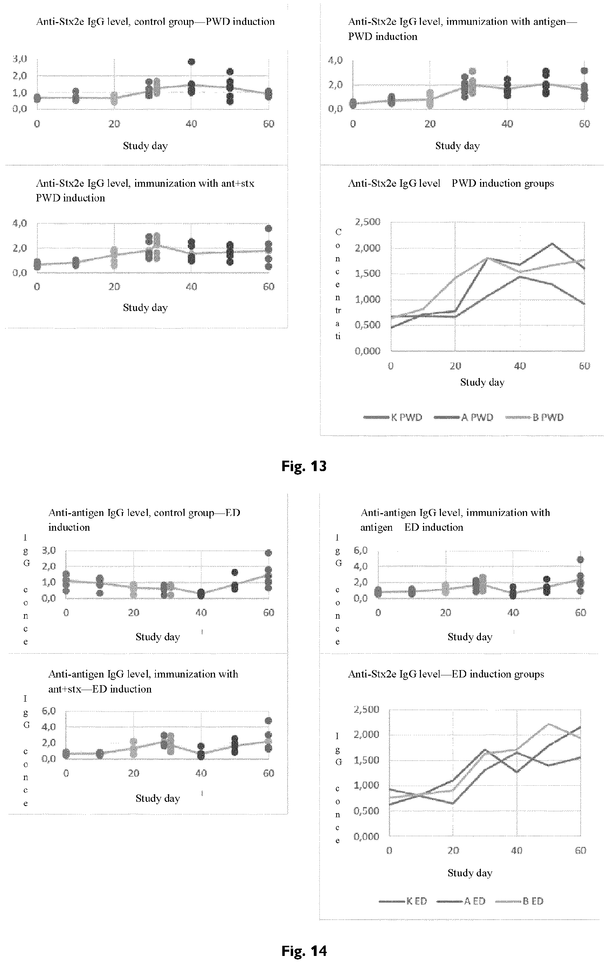

Fig. 13 shows the level of anti-Stx2eB IgG-groups with PWD induced on day 28.

In case of antibodies specific to the Stx2eB protein, there was a strange increase in their level for the control group-in this case, it could not be due to infection, since the Shiga toxin-producing strain was not administered when infected with the PWD-causing strain.

The highest level at the end of the study was recorded for both study groups. The similar levels in the groups immunized with antigen only and with antigen and protein, are a result opposite to the expected one. Immunization with antigen should not induce the production of antibodies to the Stx2eB protein.

Fig. 14 shows the level of anti-antigen IgG-groups with ED induced on day 28.

The level of antibodies specific to the antigen was higher than for the control group and obtained a similar value for the groups immunized with antigen as well as the mixture of antigen + protein.

There was a large increase in antibody levels during the study for the control group, which may have been caused by the infection, but at the end of the study, the levels in the study groups were higher than in the control groups due to immunization.

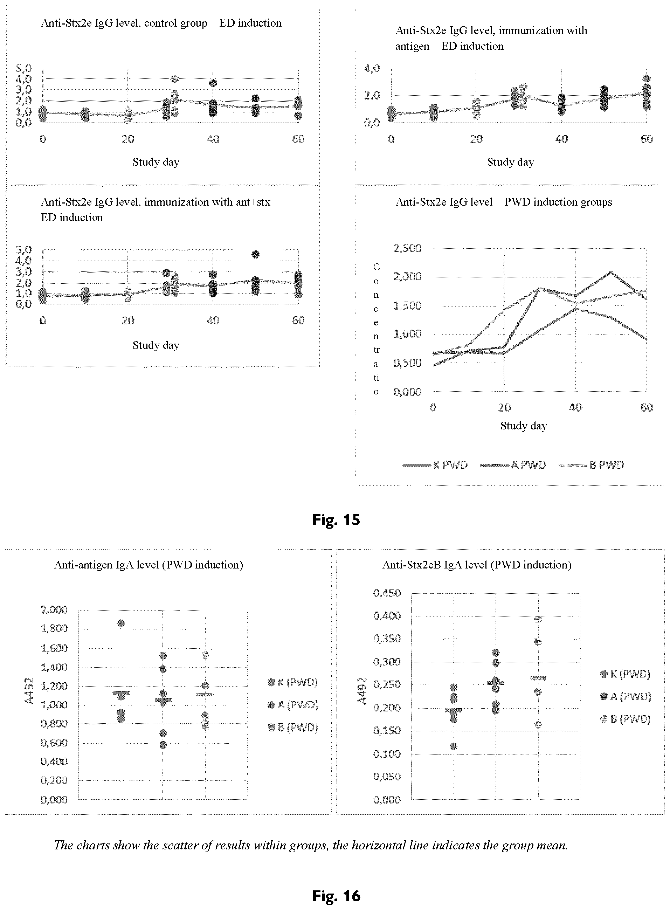

Fig. 15 shows the level of anti-Stx2eB IgG-groups with ED induced on day 28. Higher levels of antibodies were recorded for both study groups relative to the control group. This is a surprising result, since the group immunized with the antigen only should not show the presence of antibodies specific to the Stx2eB protein (on days 50 and 55 the level is even higher than for the group immunized with the protein).

Fig. 16 shows the IgA level for groups with PWD induced on day 28. IgA levels in the intestines of piglets were significantly higher for the antigen (absorbance 1-2) than for Stx2eB (0.1-0.4). This is an expected result, since the animals in these groups were not infected with the strain containing Stx2e, only group B should have higher level (it was immunized with Stx2eB protein) and it is the highest among the three groups, but not significantly higher.

High readings were recorded in the control groups because the collection of the sections took place after the end of the study (day 60) and by then the piglets in the control groups may have already developed antibodies to the strains used for infection. The scatter of results within groups is quite large.

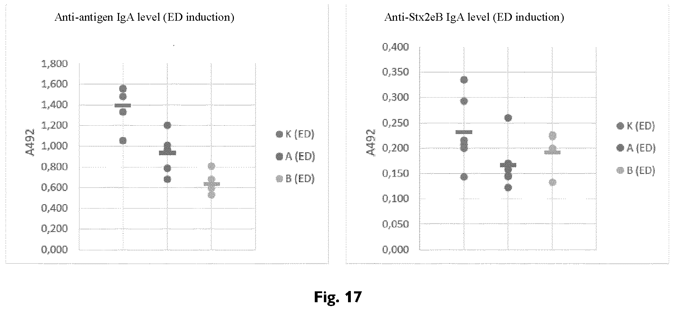

Fig. 17 shows the IgA level for groups with ED induced on day 28.

The IgA level for groups with induced ED (strain with Stx2eB)-the highest levels were recorded for the control group for both antigen- and Stx2eB protein-specific antibodies. No significant difference was recorded between the study groups.

Fig. 18 shows the level of IL-1β cytokines. Cytokine level was tested 24 h and 72 h after infection of animals with the pathogenic strain for all animals and from pooled sera for determination of pre-infection levels. The charts show the results for both time points. The charts show the scatter of results of cytokine level measurements in different groups 1 day and 3 days after infection (1-24 h after infection, 3-72 h after infection). The solid line shows the mean serum cytokine level before infection of the animals.

There is a very large scatter of results, with the largest increase observed for the group vaccinated with antigen and infected with the ED-causing strain (strain with F18 and Stx2e), and similar scatter was observed in all groups with induced PWD (F4). In case of infection with the ED-causing strain, the lowest increase was observed for the group vaccinated with the preparation containing antigen and Stx2eB protein-it is possible that the presence of antibodies to Stx2eB induced a different pathway of cytokine activation, and therefore IL-1β level in this group was much lower than in case of the control and antigen only-immunized group.

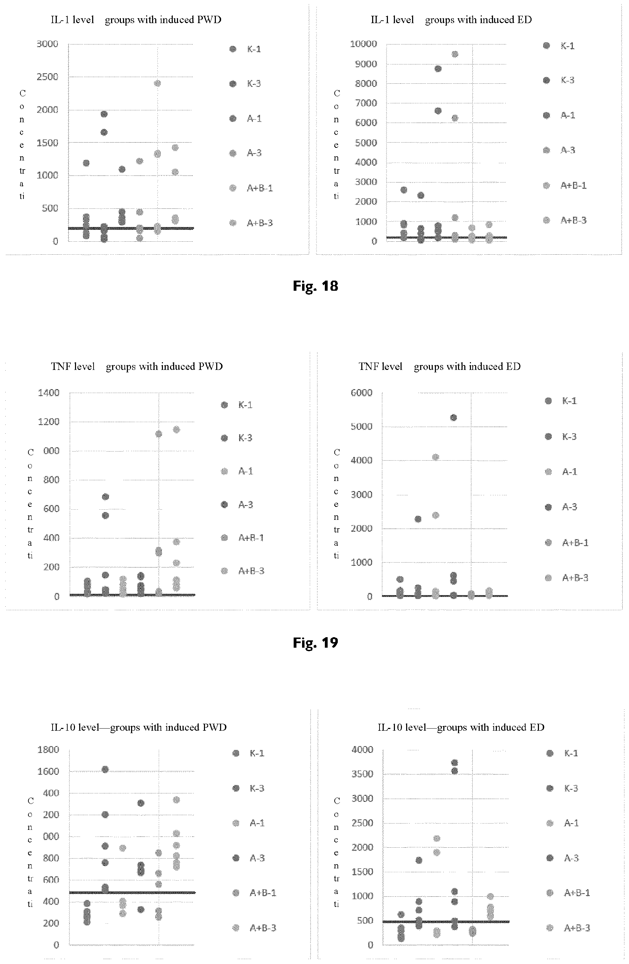

Fig. 19 shows the level of TNFα cytokines. There is a large scatter of results. Again, the smallest increase was recorded for the group immunized with the preparation containing antigens and Stx2eB protein and with induced ED (strains with F4 and Stx2e). In all groups, there was an increase in cytokine levels compared to the baseline level measured before infection (about 11 pg/mL). For most individuals, levels rose to 100-800 pg/mL, and for some even to above 4000 pg/mL.

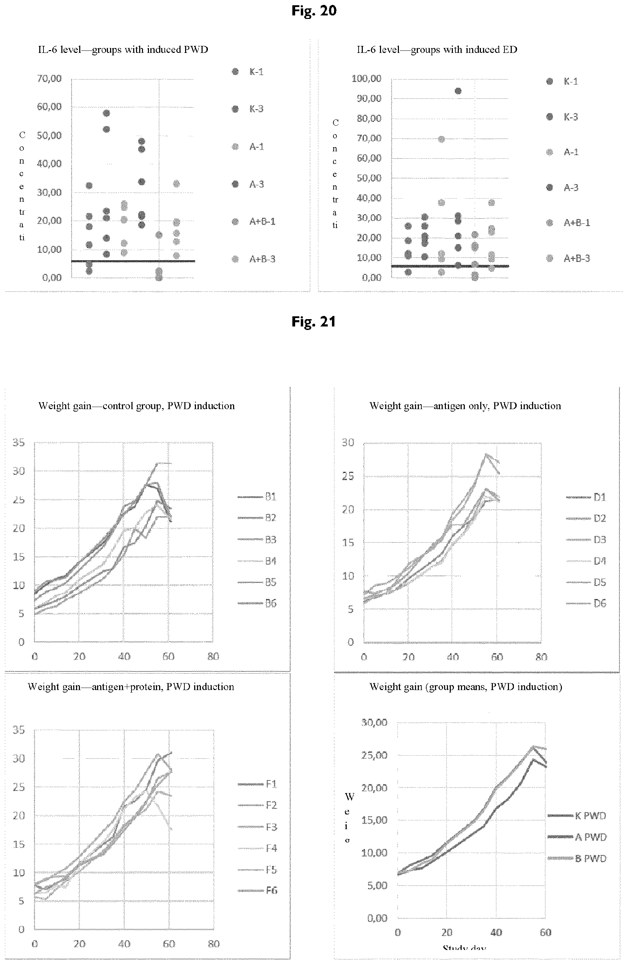

Fig. 20 shows the level of IL-10 cytokines. In case of the IL-10 (the ANTI-INFLAMMATORY cytokine), the greatest increase in its level was recorded for all groups 3 days after infection. This means that already on the 3rd day after infection the immune system inhibited pro-inflammatory processes (diarrhea on the 3rd day was still very severe, between the days 4 and 7 it stopped). The lowest increase and scatter of results were again recorded for the groups immunized with antigen and Stx2eB protein and infected with the ED-causing strain (F18, Stx2e).

Fig. 21 shows the level of IL-6 cytokines. Again, large scatter of results within the groups was observed. In all cases, there was also an increase in cytokine levels from day 1 to day 3 after infection. The levels were also higher than for the measurement of IL-6 level before infection.

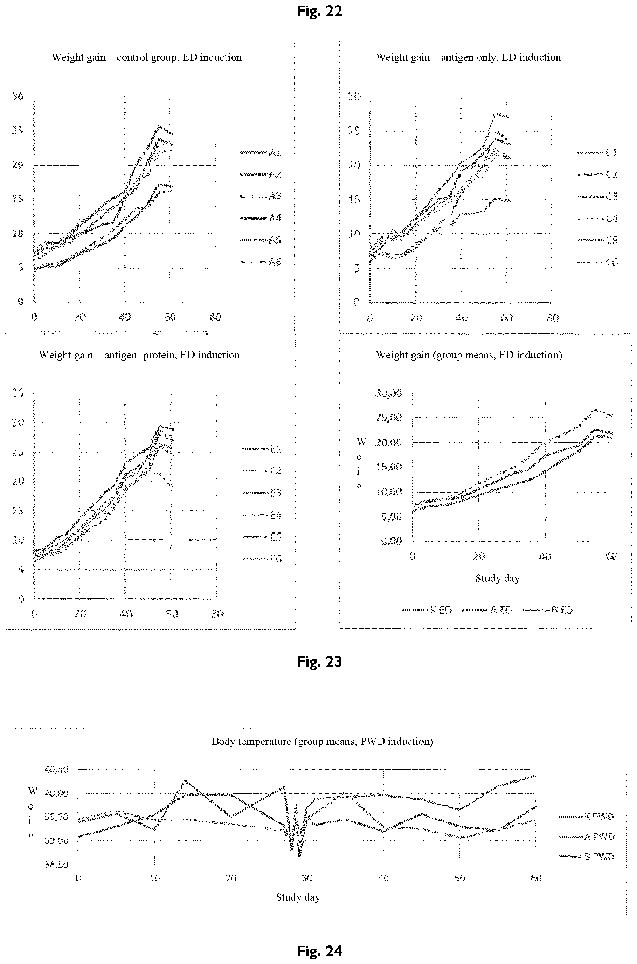

Fig. 22 shows the weight gain of the animals-PWD-induced groups. The decrease in weight between days 55 and 60 was due to withdrawal of feed before the animals were destined for slaughter for collection of intestinal sections.

Normally, between 8 and 9 weeks of age (days 56-63), the weight of piglets should be 21-25 kg.

In case of PWD infection, animals in group immunized with antigen and protein on average weighed 2 kg more than those in the control group.

Fig. 23 shows the weight gain of animals-ED-induced groups. The groups with induced ED (by Stx2eB-producing strain) show a greater difference between the control group and the study group immunized with antigen and protein. Antigen only-immunized study group showed a weight gain similar to the control group (an expected result-these animals were not immunized with the protein, which In case of ED confers key resistance to infection).

The mean weight of animals in the control group at the end of the study was 20.9 kg and in the group immunized with antigen and protein 25.3 kg (about 4.5 kg difference).

Fig. 24 shows the body temperature of the animals-PWD-induced groups. In the period after infection with the PWD-causing strain (from day 30 onward), the piglet body temperature in the control group was about 0.6-1 degree Celsius higher relative to the study groups.

There was an increase in animal body temperature after the second dose of the preparation (day 14) in the control and antigen-immunized groups. There was no increase in body temperature in the group immunized with antigen and protein.

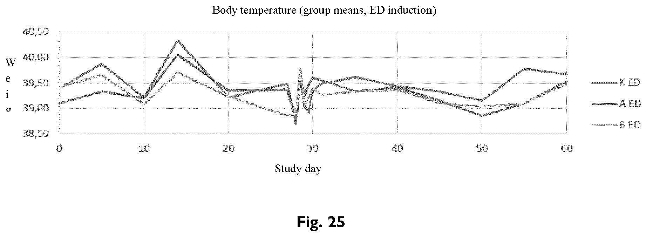

Fig. 25 shows the body temperature of the animals-ED-induced groups. There is no clear difference in piglet body temperature in ED-induced groups throughout the study. There was an increase in body temperature after the second dose of the preparation, the highest for the control group, smaller for the antigen-immunized group, and the smallest for the antigen and protein-immunized group.

### CONCLUSIONS

| **Parameter studied** | **PWD-induced groups (strain with F4)** | **ED-induced groups (strain with F18 + Stx2eB)** |
|---|---|---|
| Shedding | **There were no differences** in shedding between the study and control groups. Infection of animals was successful-all animals showed disease symptoms. The most severe diarrhea was noted in the control groups and group C (immunized with antigen only, with induced ED). | |
| | Groups immunized with the antigen only showed the fewest symptoms, those immunized with antigen and protein showed slightly loose stool 2-3 days after infection, symptoms quickly subsided, while animals in the control group showed diarrhea from infection of varying degrees of severity throughout the study period. The results indicate that the preparation helped to protect the animals from the severe symptoms that occurred in the control group. | Groups immunized with antigen and protein did not show the diarrheal symptoms that occurred in the control group and the group immunized with the antigen only. The addition of the Stx2eB protein proved effective in protecting against infection with Shiga toxin-producing *E. coli.* |
| Disease symptoms after infection | In the control group, diarrheal symptoms were observed from day 31 to 60 for most animals. In the antygen only-immunized study group, only one piglet showed severe diarrhea, and one-a mild diarrhea. In the group immunized with antigen and protein, there was a mild diarrhea after infection, which passed within 3-4 days. | Immediately after infection (day 31-35) only the control group and the group immunized with antigen only showed diarrheal symptoms. However, in all groups there were 1-2 piglets that showed diarrheal symptoms on days 37-55. |
| | **Symptoms in both control groups were significantly more severe than in the two study groups.** | |
| Anti**-antigen IgG** antibody level | **Higher IgG levels were shown for the study groups relative to the control group.** The level on days 40-60 was also increased in the control group due to the bacteria given at infection. | **Higher IgG levels were shown for the study groups relative to the control group.** The level on days 40-60 was also increased in the control group due to the bacteria given at infection. |
| Anti**-Stx2eB IgG** antibody level | IgG levels in the study groups were slightly higher relative to the control group. From day 20, there was an increase in antibodies in the control group due to the bacteria given at infection. | IgG levels in the study groups were slightly higher relative to the control group. From day 20, there was an increase in antibodies in the control group (increase of unknown origin, the animals were not exposed to the Stx2eB protein). |
| Anti-**antigen IgA** antibody level | There were no differences in IgA levels between the study groups and the control group. The level in all groups may have been high due to the immunity obtained on days 28-60 to the strain with which the animals were infected. | The highest level recorded was for the control group. The level in all groups may have been high due to the immunity obtained on days 28-60 to the strain with which the animals were infected. |
| Anti-**Stx2eB IgA** antibody level | The highest level was recorded for the group immunized with antigen and protein. Levels in all groups were much lower than for antigen, there were no significant differences between groups. | The highest level was recorded for the control group; there were no significant differences between groups. |
| Cytokines | There was an increase in cytokine levels compared to levels before infection. There was also an increase in the level of the anti-inflammatory cytokine IL-10 on day 3 after infection, indicating an anti-inflammatory effect. The smallest fluctuations in cytokine levels were observed for the groups immunized with antigen and protein-this may indicate that the animals have acquired immunity and their bodies fought the infection with less effort and in the control groups a much higher cytokine release was required to suppress the infection. | |
| Body weight | The mean body weight in the group immunized with antigen and protein was about 2 kg higher than for the control group. | For the group immunized with antigen and protein, a better weight gain (by 4.4 kg) was shown compared to the control group and the antigen only-immunized group. **Again, this indicates that the addition of Stx2eB protein had** a **positive effect on piglet health during the study.** |
| Temperature | The piglet body temperature in the control group since infection was higher than in the study groups, **indicating that the vaccine protected the animals from severe inflammation.** | There were no differences in piglet body temperature between the groups. |

The applied preparation (both versions) showed a protective effect compared to no application (control groups). The preparation consisting of an antigen and a protein showed a protective effect in case of both PWD and ED induction. The above conclusions are mainly indicated by:
→ The results of disease symptom observation in animals during the study (especially after infection);
→ Measurement of animal weight (the animals from the study groups were on average 2-4.5 kg heavier at the end of the study than the control animals)-for economic reasons, this is a key parameter in pig farming (farmers mainly care about the best possible weight gain of animals). Animals between 56 and 63 days of age should weigh between 21 and 25 kg, while in the study in the control groups the mean weight was 21-23 kg, and in the groups immunized with antigen and protein it was 25.3-30 kg. Since in healthy piglets the norm is 21-25 kg, and in the present study the piglets, despite suffering infection with a highly immunogenic strain, reached the upper limit of the parameter, the preparation can be considered as having a protective effect and a positive impact on animal health;
→ The anti-antigen IgG antibody levels (higher in the study groups than in the control group).

With each preparation dose (days 0 and 14), the piglets also received a mixture of IgY antibodies per os for passive protection against E coli infection. On these days, and until infection (day 28), there was a significant decrease in coliform shedding in swabs taken from the animals. This may be related to the preventive administration of IgY antibodies.

The administration of the preparation did not cause more severe adverse symptoms than the placebo administration to the control groups, nor were there any symptoms directly (injection site reaction) or indirectly (e.g., diarrhea, shock, lethargy, etc.) related to the administration of the preparation. The body temperature of animals immunized with the preparation increased less than that of control animals immunized with placebo.

### Round II studies on piglets

First dose: 100 µg of Stx2eB + 10⁹ CFU of 4 inactivated strains (A4, V5, K2, and K3) in a ratio of 1:1:1:1 + Montanide Gel02 adjuvant (20%)
Second dose: 500 µg of Stx2eB + 5×10⁹ CFU of 4 inactivated strains (A4, V5, K2, and K3) in a ratio of 1:1:1:1.

All study animals showed an increase in body weight over time. When comparing the vaccinated groups, a greater increase in weight was shown in the PWD group (better results were also obtained in this group when body temperature was compared). In the vaccinated group (which was also given IgY antibodies for several days), the weight gain averaged 7.7 kg.

When the anti-ED vaccine was administered, the comparison of the first and second rounds of studies on piglets (up to 21 days of age, comparing animals with similar initial weights) showed no significant differences in the mean piglet body weight. In both rounds, large fluctuations in body weight can be observed (larger than 4 kg), however, these are probably changes due to individual characteristics. In case of PWD, a larger weight gain of about 1-2 kg was observed in the second round of the study compared to the first round.

## Claims

1. A vaccine for use in the prevention and/or treatment of colibacterioses in piglets, **characterized in that** it contains:
- a mixture of four strains of whole inactivated *Escherichia coli* bacteria, strains obtained from animals with symptoms of either post-weaning diarrhea or edema disease, with the following characteristics:
| **Feature/strain** | **A4** | **V5** | **K2** | **K3** |
|---|---|---|---|---|
| Surface antigens (serologically determined) | O:139 | O:149 | O:139, F:4 | O:139 |
| Genes, surface antigens (determined by PCR) | F18, STb | F18, STb, LT, AIDA, F5, and F41 | F4, STb, F5 | Stx2e, STb, F5 |
| Antibiotic resistance | gentamicin, rifampicin, cefalotin | rifampicin, cefalotin | rifampicin, chloramphenicol | rifampicin, chloramphenicol |
and
- Stx2eB protein with sequence **SEQ ID NO: 1** and/or **SEQ ID NO: 3** and/or **SEQ ID NO: 5.**

2. The vaccine according to claim 1, which contains additives such as a pharmaceutically or veterinary acceptable carrier such as saline and an adjuvant.

3. The vaccine according to claim 1 or 2, **characterized in that** it contains a mixture of four strains of whole inactivated *Escherichia coli* bacteria mixed in a ratio of 1:1:1:1.

4. The vaccine according to claim 1 or 2 or 3, **characterized in that** it contains:
1-5×10⁹ CFU of a mixture of four strains of whole inactivated *Escherichia coli* bacteria mixed in a ratio of 1:1:1:1.
and
50-500 µg of Stx2eB proteins with the sequence **SEQ ID NO: 1** and/or **SEQ ID NO:** 3 and/or **SEQ ID NO: 5.**

5. The vaccine according to any of the claims 1-4, for administration by injection.

6. A method for obtaining a vaccine as defined in any of the claims 1-5, **characterized in that** it includes steps in which:
- Stx2eB protein is produced;
- a mixture of four strains of whole inactivated *Escherichia coli* bacteria is prepared, followed by
- mixing the produced Stx2eB protein with the mixture of four strains of whole inactivated *Escherichia coli* bacteria;
wherein
- the Stx2eB protein is produced by culturing an expressive *E. coli* strain transformed with a gene encoding the Stx2eB protein or its modified version, and
- the mixture of inactivated strains is obtained by inactivating the bacterial culture with 10% formalin.

7. A use of the vaccine defined in claims 1-5 for production of an immunizing and/or therapeutic preparation against colibacterioses in piglets.

8. The immunizing and/or treatment preparation against colibacteriosis in piglets, **characterized in that** it comprises:
- the vaccine as defined in claims 1-5 for administration by injection, and
- chicken IgY antibodies to *E. coli* strains having O:139, O:149, F:4, F18, F5 antigens, for oral administration.

9. The preparation according to claim 8, **characterized in that** it contains additives such as a pharmaceutically or veterinary acceptable carrier such as saline, an adjuvant, a preservative such as thimerosal.

10. The preparation according to claim 8 or 9, **characterized in that** the chicken IgY antibodies to *E. coli* strains having O:139, O:149, F:4, F18, F5 antigens were obtained by immunizing chickens with four *Escherichia coli* strains with the following characteristics:
| **Feature/strain** | **A4** | **V5** | **K2** | **K3** |
|---|---|---|---|---|
| Surface antigens (serologically determined) | O:139 | O:149 | O:139, F:4 | O:139 |
| Genes, surface antigens (determined by PCR) | F18, STb | F18, STb, LT, AIDA, F5, and F41 | F4, STb, F5 | Stx2e, STb, F5 |
| Antibiotic resistance | gentamicin, rifampicin, cefalotin | rifampicin, cefalotin | rifampicin, chloramphenicol | rifampicin, chloramphenicol |

11. The preparation according to claim 8 or 9 or 10, **characterized in that** the chicken IgY antibodies obtained by immunization of chickens with four strains of *Escherichia coli* with the characteristics defined in claim 10 are combined in a ratio of 1:1:1:1.

12. The preparation according to any of the claims 8-11, **characterized in that** it contains:
1-5×10⁹ CFU of a mixture of four strains of whole inactivated *Escherichia coli* bacteria mixed in a ratio of 1:1:1:1 and 50-500 µg of Stx2eB proteins with the sequence **SEQ ID NO: 1** and/or **SEQ ID NO: 3** and/or **SEQ ID NO: 5** in the form of a vaccine for administration by injection and chicken IgY antibodies to E. coli strains having O:139, O:149, F:4, F18, F5 antigens, for oral administration in the amount of 15-500 mg.

13. The preparation according to any of the claims 8-12, **characterized in that** it is administered in two doses at an interval of either 7 or 14 days.

14. The preparation according to any of the claims 8-13, **characterized in that** it is administered according to the following scheme:
| Administration | Age of the animal | Composition of the injection dose | Composition of the oral dose |
|---|---|---|---|
| First dose | The 18th day of life of the piglet | 10⁹ CFU of a mixture of 4 antigens mixed in a ratio of 1:1:1:1, 0.25x10⁹ of each strain and 50 µg of Stx2eB protein | 15 mg of IgY antibodies mixed in a ratio of 1:1:1 :1, 3.75 mg of each type of antibody |
| Second dose | The 32nd day of life of the piglet | 5x10⁹ CFU of a mixture of 4 antigens mixed in a ratio of 1:1:1:1, 1.25x10⁹ of each strain and 100 µg of Stx2eB protein | 15 mg of IgY antibodies mixed in a ratio of 1:1:1 :1, 7.5 mg of each type of antibody |

15. The preparation according to any of the claims 8-14, **characterized in that** the chicken IgY antibodies are administered daily in an amount of 15-500 mg/day.
